# FASCICULE DE BREVET EUROPEEN

(11) **EP 2 719 425 B1**
(45) Date de publication et mention de la délivrance du brevet: **08.04.2015**
(21) Numéro de dépôt: 13172889.1
(22) Date de dépôt: 19.06.2013
(51) Int. Cl.: A61N 1/362, A61N 1/375, A61N 1/05

(54) **Sonde de stimulation cardiaque implantable le long de la paroi du septum interventriculaire et/ou de la paroi libre du ventricule gauche**
Herzschrittmachersonde, die entlang der interventrikulären Septumswand und/oder der freien Wand des linken Ventrikels implantiert werden kann
Implantable cardiac stimulation probe along the ventricular septal wall and/or the left ventricle free wall

(30) Priorité: 12.10.2012 FR 1259761
(43) Date de publication de la demande: 16.04.2014
(73) Titulaire: Sorin CRM SAS, 92140 Clamart Cedex (FR)
(72) Inventeur: Ollivier, Jean-François, 91190 Villiers Le Bacle (FR)
(74) Mandataire: Dupuis-Latour, Dominique

(56) Documents cités:
- EP-A1- 2 384 784
- US-A1- 2005 080 470
- US-A1- 2005 209 668
- US-A1- 2006 106 442
- US-A1- 2006 258 978
- US-A1- 2008 294 229
- US-A1- 2011 066 057
- US-A1- 2012 136 423
- US-B2- 6 718 206
- US-B2- 7 027 876

## Description

L'invention concerne les sondes intracardiaques de stimulation du ventricule gauche.

L'invention se situe dans le contexte général des "dispositifs médicaux implantables actifs" tels que définis par la directive 90/385/CEE du 20 juin 1990 du Conseil des communautés européennes, notamment les implants permettant de surveiller en continu le rythme cardiaque et délivrer si nécessaire au coeur des impulsions électriques de stimulation, de resynchronisation ou de défibrillation.

On fera principalement référence dans la présente description à des sondes intracardiaques "de stimulation", c'est-à-dire destinées à la délivrance d'impulsions à basse énergie utilisées pour les thérapies antibradycardiques ou de resynchronisation. Mais l'invention s'applique également aux sondes intracardiaques de cardioversion/défibrillation destinées à délivrer au coeur des chocs électriques de haute énergie pour tenter de mettre fin à une tachyarythmie. Sauf indication contraire, les termes génériques de "sonde (ou électrode) de stimulation", ou "de stimulation/défibrillation" viseront tout type de sonde utilisable à ces fins, quels que soient le type et le niveau d'énergie électrique délivré.

Pour la stimulation du ventricule droit, il suffit d'implanter une sonde endocavitaire par le réseau veineux périphérique droit. En revanche, pour stimuler le ventricule gauche, la situation est plus complexe.

Des solutions très diverses ont été proposées à cet effet : sonde introduite dans le réseau coronarien via l'oreillette droite puis l'ostium du sinus coronaire, sonde introduite dans le ventricule droit et positionnée contre la paroi du septum interventriculaire, ou encore perçage du septum puis introduction d'une sonde traversant ce septum jusqu'à ce que cette dernière vienne en contact direct avec une paroi interne du ventricule gauche. Une autre technique, plus difficile à mettre en oeuvre et présentant un caractère beaucoup plus invasif, consiste à implanter des électrodes épicardiques sur la paroi externe du myocarde, en un ou plusieurs sites appropriés disposés face à la cavité du ventricule gauche. La mise en place d'une telle sonde est cependant une opération très lourde, nécessitant généralement une anesthésie générale et le recours à des techniques chirurgicales très invasives. Pour cette raison, cette solution est souvent considérée comme une solution de dernier recours, en cas d'échec d'implantation via le sinus coronaire. En outre, les performances électriques sont souvent médiocres, et il est très difficile de modifier le site d'implantation initialement choisi et si besoin d'explanter ultérieurement la sonde. L'invention repose sur un autre principe, dont l'idée de base consiste à injecter un ou plusieurs microcâbles dans l'épaisseur de la paroi du septum interventriculaire, et/ou respectivement dans l'épaisseur de la paroi libre du ventricule gauche, sous la surface de cette paroi et sur la longueur de celle-ci s'étendant entre la région de l'apex et la région auriculaire, c'est-à-dire sur la majeure partie de la longueur de ces parois. Le microcâble est en principe destiné à rester sous la surface de la paroi, sans déboucher à l'intérieur de la cavité, ni non plus à l'extérieur du myocarde si ce n'est au point de liaison à un corps de sonde relié au générateur d'impulsions. Les microcâbles suivront ainsi approximativement le trajet de la branche gauche du faisceau de His, qui est une ligne de conduction électrique rapide interne du myocarde s'étendant le long du septum interventriculaire au voisinage du ventricule gauche, puis remontant le long de la paroi libre de ce même ventricule gauche.

La branche gauche du faisceau de His joue un rôle prépondérant dans le mécanisme de resynchronisation du ventricule gauche, en constituant une voie de conduction rapide (de l'ordre de 4 m/s) à partir de laquelle la conduction de l'onde de dépolarisation s'amorce, et se propage de proche en proche à une vitesse sensiblement plus lente (0,4 m/s) dans le reste des tissus du myocarde. Préserver, restaurer ou améliorer cette fonction a pour effet de contribuer sensiblement à l'amélioration des performances hémodynamiques.

L'implantation d'un microcâble à cet endroit permet de rétablir une ligne de conduction complète, proche du trajet naturel au sein du myocarde. Cette ligne de conduction sera dotée d'un ou plusieurs points de stimulation à partir desquels pourra s'amorcer une conduction naturelle, et sans délai même par exemple en cas de bloc gauche local.

Cette approche selon l'invention, consistant à reconstituer artificiellement la branche gauche du faisceau de His et à y appliquer une stimulation électrique en de multiples points, s'oppose aux systèmes classiques de stimulation, qui visent tous à cibler quelques points de stimulation dont l'efficacité peut être vite altérée si les tissus environnants ne sont pas viables.

De plus, comme on le verra, du point de vue du geste chirurgical les microcâbles peuvent être injectés par une approche conventionnelle de type sub-xiphoïdienne (passage d'une aiguille sous la région inférieure du sternum), peu invasive et ne nécessitant que des instruments simples.

Le US 2008/0294229 A1 décrit une sonde de stimulation permettant notamment la stimulation du ventricule gauche par implantation dans l'épaisseur de la paroi du septum interventriculaire ou bien dans l'épaisseur de la paroi libre du ventricule gauche, sous la surface et le long de cette paroi entre la région de l'apex et la région auriculaire..

Le US 2005/0080470 A1 décrit une technique particulière d'implantation d'une sonde de détection/stimulation par voie transthoracique. Cette technique est toutefois particulièrement invasive, en raison des calibres élevés des instruments utilisés pour traverser le thorax.

Plus précisément, l'invention propose un ensemble de sonde intracardiaque de stimulation du ventricule gauche, apte à être combiné avec un générateur d'un dispositif médical implantable actif pour la délivrance d'impulsions de stimulation et/ou de resynchronisation et/ou de défibrillation cardiaque.

Cet ensemble comporte une sonde intracardiaque et un accessoire d'implantation de la sonde dans les tissus d'une région interne du myocarde.

D'une manière connue, notamment d'après le US 2008/0294229 A1 précité, la sonde est destinée à être implantée dans les tissus d'une région interne du myocarde, par pénétration dans l'épaisseur de la paroi du septum interventriculaire ou bien dans l'épaisseur de la paroi libre du ventricule gauche, sous la surface et le long de cette paroi entre la région de l'apex et la région auriculaire. Plus précisément, cette sonde est une microsonde constituée dans sa partie active, distale par un microcâble comportant un coeur électriquement conducteur revêtu d'une couche électriquement isolante, la partie active comprenant une série de zones dénudées du microcâble formant électrodes de stimulation électriquement reliées ensemble.

De façon caractéristique, le diamètre du microcâble est d'au plus 1 French (0,33 mm), et son extrémité distale libre est repliée sur elle-même. Par ailleurs, l'accessoire d'implantation comporte une aiguille avec une extrémité libre pointue de ponction et une extrémité opposée montée sur un embout de préhension, cette aiguille étant au moins dans sa partie distale une aiguille creuse comportant une lumière interne débouchante. La partie repliée de l'extrémité distale du microcâble est insérée dans la lumière interne de l'aiguille, et la partie non repliée du microcâble s'étend contre la surface extérieure de l'aiguille le long de celle-ci jusqu'à l'embout de préhension. L'accessoire d'implantation comporte en outre des moyens libérables de maintien du microcâble, pour le support et la rétention du microcâble sur la longueur de l'aiguille entre l'extrémité de ponction et l'embout de préhension.

De cette manière, il est possible d'implanter la microsonde par pénétration simultanée de l'aiguille et du microcâble porté par cette aiguille, dans l'épaisseur de la paroi du septum interventriculaire ou de la paroi libre du ventricule gauche.

Selon diverses caractéristiques subsidiaires avantageuses :
- la partie distale de l'aiguille comporte un canal prolongeant la lumière interne et apte à permettre la mise en place et le retrait de l'extrémité repliée du microcâble ;
- à l'endroit du bord proximal du canal, le débouché de la lumière centrale de l'aiguille n'est pas affuté, de manière à éviter tout endommagement de la partie repliée du microcâble à l'endroit de la commissure de celle-ci ;
- la longueur de la partie active du microcâble comprenant la série de zones dénudées formant électrodes est comprise entre 50 et 150 mm ;
- la longueur de la partie repliée de l'extrémité distale du microcâble est comprise entre 2 et 5 mm ;
- l'embout comprend un plot élastique, notamment un plot de matériau silicone comportant une fente longitudinale de clipsage du microcâble, formant moyen libérable de maintien du microcâble, de manière à permettre le maintien de celui-ci à l'état tendu entre le plot et le débouché de la lumière interne de l'aiguille ;
- le coeur électriquement conducteur du microcâble comprend une structure composite avec une pluralité de brins toronnés ensemble, dont au moins certains sont des brins incorporant une âme en matériau radio-opaque de type platine-iridium ou tantale enveloppée dans une gaine en matériau mécaniquement endurant de type NiTi ou alliage de type MP35NLT, ou inversement ;
- le diamètre externe de l'aiguille est compris entre 0,2 et 0,5 mm ;
- l'ensemble comprend en outre des moyens d'ancrage du microcâble à une surface du myocarde ;
- ces moyens d'ancrage comprennent une capsule à vis hélicoïdale, pourvue de moyens de solidarisation au microcâble dans une région axiale de la capsule ;
- la surface extérieure de la capsule à vis peut être conformée en un tambour d'enroulement apte à recevoir une longueur excédentaire du microcâble, ou bien la capsule comprend un organe élastique avec un perçage axial apte à recevoir l'aiguille pourvue du microcâble à proximité de l'embout de préhension ;
- les moyens d'ancrage peuvent également comprendre un point de colle d'un adhésif chirurgical biocompatible ;
- la surface totale exposée des zones dénudées de la microsonde est d'au plus 6 mm².

On va maintenant décrire un exemple de mise en oeuvre de l'invention, en référence aux dessins annexés où les mêmes références numériques désignent d'une figure à l'autre des éléments identiques ou fonctionnellement semblables.
La Figure 1 est une vue en coupe schématique du myocarde, montrant les différentes cavités ainsi que les principales voies de conduction électrique.
La Figure 2 illustre un ensemble de sonde selon l'invention, dans une configuration prête à l'implantation des microcâbles.
La Figure 3 illustre, isolément, l'aiguille d'implantation de l'ensemble de la Figure 2.
La Figure 4 montre le détail de la pointe de l'aiguille de la Figure 3.
La Figure 5 montre des détails de la pointe et du corps de l'aiguille de la Figure 2, avec le microcâble en configuration prête à l'implantation.
La Figure 6 est une vue en coupe de l'extrémité distale de l'aiguille et du microcâble illustrés Figures 2 et 5.
La Figure 7 illustre, isolément, une capsule d'ancrage à visser sur l'épicarde au débouché du microcâble.
La Figure 8 illustre la capsule d'ancrage de la Figure 7, mise en position sur l'aiguille de l'ensemble prêt à l'implantation illustré Figure 2.
La Figure 9 est un schéma de la cage thoracique et du coeur, illustrant la voie d'accès pour l'injection des microcâbles.

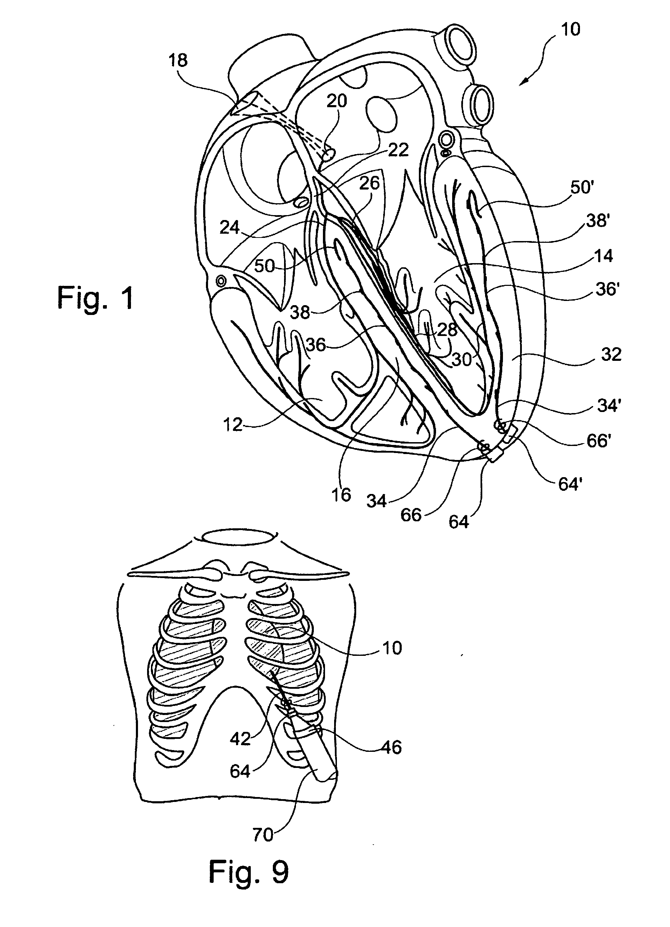

La Figure 1 représente schématiquement, en coupe, le myocarde 10 avec le ventricule droit 12 et le ventricule gauche 14 séparés par le septum interventriculaire 16. Celui-ci présente une épaisseur typique de l'ordre de 10 à 15 mm et constitue une partie significative de la masse cardiaque.

Les ondes de dépolarisation qui prennent naissance dans le noeud sino-atrial 18 sont transmises au noeud atrioventriculaire 20 puis au faisceau de His 22 qui se divise en deux branches s'étendant le long du septum 16, avec une branche droite 24 dans la région de la paroi septale droite et une branche gauche 26 dans la région de la paroi septale gauche 28.

En particulier, la branche gauche 26 constitue une ligne de conduction électrique longitudinale rapide, avec une vitesse de l'ordre de 4 m/s. Cette branche gauche s'étend le long de la paroi gauche 28 du septum interventriculaire 16 le long de celle-ci jusqu'à la région de l'apex, puis remonte en 30 le long de la paroi libre 32 du ventricule gauche, approximativement jusqu'à la région de l'oreillette.

L'idée de base de l'invention consiste à implanter une ou, très préférentiellement, plusieurs microsondes 34, 34', dont les extrémités actives respectives sont constituées de microcâbles 36, 36' pourvus chacun d'une ou plusieurs d'électrodes de stimulation 38, 38', de préférence un très grand nombre d'électrodes de très petite dimension chacune.

L'invention n'est d'ailleurs pas limitée à la configuration décrite où l'on a illustré un ensemble à deux microsondes : en effet, il est possible d'envisager l'implantation non plus d'une, mais de plusieurs microsondes dans la paroi du septum interventriculaire et/ou dans la paroi libre du ventricule gauche. Une telle multiplication des microsondes conduira à former autour du ventricule gauche un "maillage" distribuant dans le muscle cardiaque un réseau de microélectrodes elles-mêmes portées par une pluralité de microcâbles rayonnant à partir de l'apex.

Les microcâbles avec leurs électrodes permettent de reconstituer artificiellement une ligne de conduction parallèlement à la branche gauche du faisceau de His et d'exciter en une multiplicité de points le ventricule gauche tant du côté de la paroi septale que du côté de la paroi libre.

De préférence, la surface totale des électrodes d'une microsonde ne dépasse pas 6 mm², valeur du même ordre que la surface active d'une sonde endocavitaire standard. Le très faible diamètre du microcâble (typiquement 0,1 mm) permet de réaliser des électrodes sur une longueur cumulée de 20 mm sans dépasser la valeur précitée de 6 mm² de surface dénudée : si l'on réduit la longueur d'une électrode individuelle à 0,5 mm, ceci laisse un potentiel de quarante électrodes réparties le long d'un même microcâble, donc une multiplication très importante des points de stimulation, qui en outre sont directement en contact avec les tissus, donc avec une excellente qualité de transmission de l'impulsion de stimulation de l'électrode aux tissus.

Dans une variante, le microcâble peut toutefois être complètement dénudé dans sa partie active distale, configuration correspondant donc à une électrode unique de grande longueur.

Le microcâble peut comprendre un coeur constitué d'une pluralité de brins composites toronnés ensemble, avec par exemple un brin central entouré de six brins périphériques. Chaque brin composite est lui-même constitué d'un fil d'âme central en alliage de type MP35NLT, enveloppé d'une gaine en platine iridié (pour la radio-opacité et la biostabilité). Ces différents fils sont disponibles dans le commerce, par exemple auprès de la société Fort Wayne Metals Inc., Fort Wayne, USA, et sont utilisés dans le domaine médical.

Le microcâble est revêtu d'une fine couche d'isolement, de l'ordre de 25 µm d'épaisseur. Les caractéristiques requises pour cette couche sont : résistance à la fatigue ; isolement électrique ; biocompatibilité à long terme ; biostabilité ; et possibilité de transformation et mise en oeuvre compatible avec le conducteur du câble de coeur. Pour réaliser cette couche d'isolement, on privilégiera les matériaux à forte inertie chimique comme les polymères fluorés, qui présentent également une très bonne qualité d'isolation. Parmi ces composés, on peut citer en particulier l'ETFE (éthylène tétrafluoroéthylène). Les procédés de réalisation de la couche d'isolement sur le câble de coeur sont, par exemple, la co-extrusion sur le conducteur ou l'échauffement d'un tube thermorétractable.

La partie active du microcâble comporte dans l'exemple illustré une pluralité de parties dénudées formant une succession d'électrodes individuelles, constituant ensemble un réseau d'électrodes reliées en série permettant de multiplier les points de stimulation. Ceci permet de multiplier les possibilités de points de contact avec les tissus et d'assurer ainsi une diffusion multizone de l'énergie de stimulation en plusieurs points du ventricule gauche. Les électrodes sont formées par exemple par ablation plasma de la couche de parylène. Pour améliorer les performances électriques, ces zones peuvent être en outre revêtues par exemple de nitrure de titane.

Du fait de la faible surface active cumulée, on bénéficie ainsi des avantages d'une sonde dite à "haute densité de courant", en termes à la fois d'efficacité physiologique de la stimulation et de moindre consommation d'énergie - ceci tout en maximisant l'étendue de la zone de contact physique, donc électrique, avec les tissus excitables. Par ailleurs, la localisation myocardique des électrodes limite les risques de stimulations phréniques.

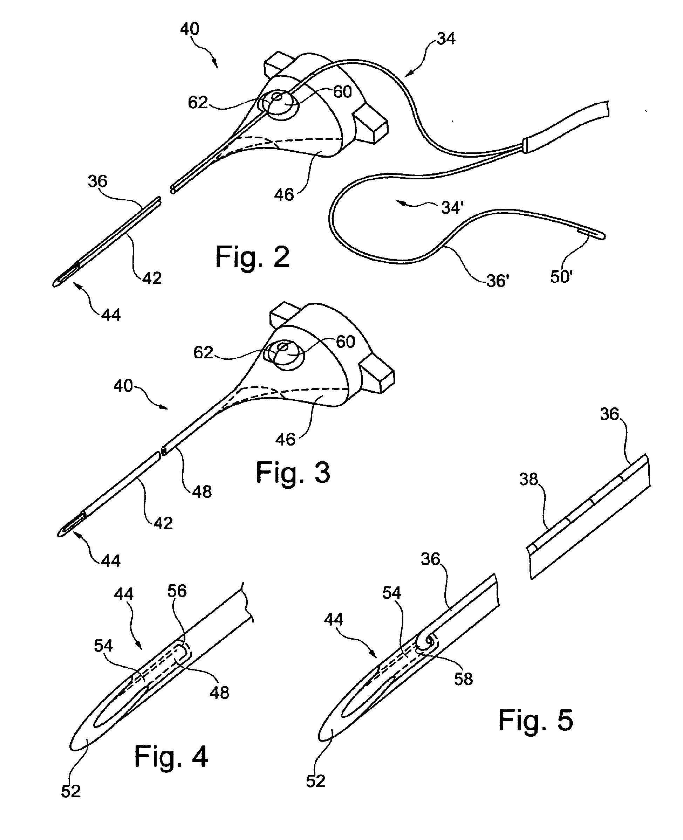

On va maintenant exposer, en référence aux Figures 2 à 9, la manière d'implanter ces microcâbles 36, 36' au moyen d'un accessoire en forme d'aiguille hypodermique permettant d'introduire directement, ou "injecter" le microcâble dans les tissus du myocarde, et ce par un geste chirurgical comparable à la manipulation d'une seringue pourvue d'une aiguille hypodermique.

L'accessoire d'implantation, référencé 40, comprend une aiguille creuse 42 comportant une extrémité distale pointue 44, et montée à son extrémité proximale opposée sur un embout de préhension 46. L'embout de préhension 46 est avantageusement un embout de connexion de type *Luer lock* à verrou permettant le montage direct sur une seringue qui fera office de poignée de manipulation du système avec, en outre, possibilité d'injecter un produit de contraste ou du sérum physiologique.

L'aiguille présente un diamètre typique de 0,2 à 0,5 mm et peut être réalisée en un matériau superélastique tel que le nitinol, pour augmenter sa résistance à la plicature.

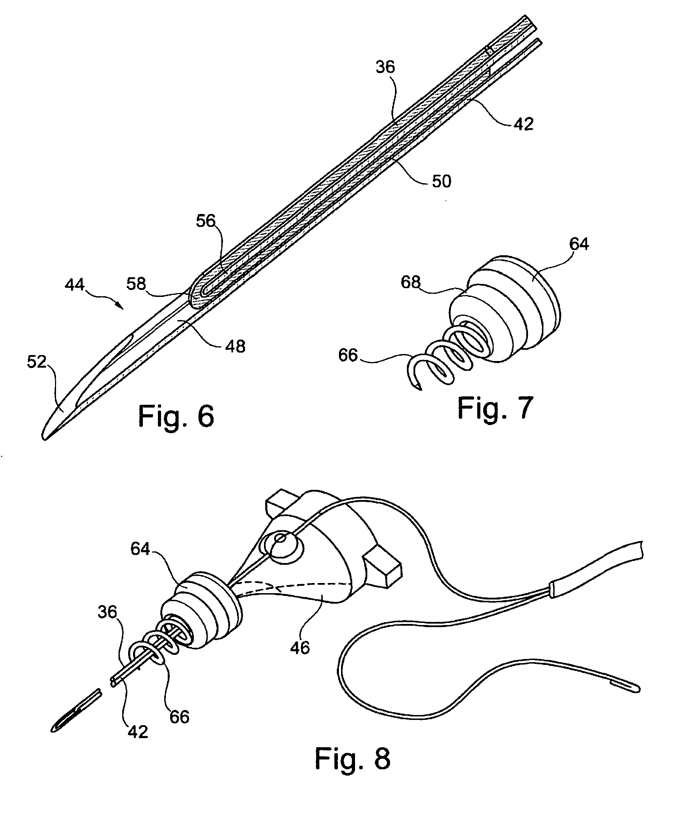

Cette aiguille 42 a pour fonction d'assurer un support mécanique au microcâble 36 qui, du fait de son extrême finesse (typiquement 0,1 mm, de l'ordre de l'épaisseur d'un cheveu), ne pourrait pas être directement implanté dans la paroi du myocarde. L'extrémité du microcâble 36 ou 36' est repliée sur elle-même respectivement en 50, 50' sur environ 2 à 5 mm de manière à former un crochet de fixation introduit, comme on peut le voir sur les Figures 5 et 6, dans la lumière interne 48 de l'aiguille 42.

La partie distale 44 de l'aiguille 42 présente une extrémité affutée 52 pour permettre une découpe fine des tissus sans les déchirer, minimisant ainsi leur endommagement et garantissant une cicatrisation rapide. Cette région comporte également un canal 54 prolongeant la lumière interne 48 pour permettre la mise en place et le retrait de l'extrémité repliée 50 du microcâble 36. Le bord proximal 56 de cette découpe n'est, en revanche, pas affuté pour éviter tout endommagement de la commissure 58 du microcâble.

Le corps de l'aiguille est conformable manuellement par le médecin afin de s'adapter à la morphologie des parois à ponctionner.

Le microcâble 36, accroché à l'aiguille à son extrémité distale par l'extrémité repliée 50, est maintenu légèrement sous tension le long de cette aiguille, étant bloqué en translation par clipsage dans un plot élastique 60 prévu sur l'embout de préhension 46. Le plot élastique 60 est par exemple un plot de silicone comprenant une fente longitudinale 62 dans laquelle le microcâble 36 est clipsé en force. Afin de renforcer la liaison temporaire entre aiguille et microcâble, ce dernier peut être enroulé autour du corps de l'aiguille avant d'être clipsé dans la fente 62 du plot silicone 60. Comme illustré Figure 2, au-delà de ce plot 60 le microcâble se prolonge en direction proximale et rejoint l'autre microcâble 36'. Les deux microcâbles sont réunis dans un corps de sonde traditionnel, muni à son autre extrémité (extrémité proximale du corps de sonde, non illustrée) d'un connecteur destiné à être relié à un boitier de générateur, selon des procédures connues.

Il est important de noter que la méthode et l'outil d'implantation précédemment décrits permettent de disposer d'une sonde "prête à l'emploi" et "d'emploi facile" ne nécessitant :
- ni une intervention du médecin pour établir une connexion microcâble/corps de sonde,
- ni un pelage de l'aiguille de ponction.

Du point de vue électrique, les deux microcâbles 36, 36' peuvent être soit reliés ensemble, soit reliés à des pôles distincts du générateur, permettant dans ce dernier cas de programmer un délai entre la stimulation des deux parois (septale et libre) du ventricule gauche. Dans le cas d'un grand nombre de microcâbles, l'extrémité du corps de sonde peut être dotée d'un multiplexeur électronique permettant de contrôler la distribution d'énergie (séquencement, type de signal, niveau d'énergie) sur les différents microcâbles.

Par ailleurs, il est possible d'envisager l'utilisation non seulement de microcâbles monopolaires comme ceux que l'on vient de décrire (c'est-à-dire dont toutes les électrodes sont électriquement reliées ensemble), mais également de microcâbles multipolaires, par exemple constitués par une pluralité de microcâbles élémentaires isolés individuellement et toronnés ensemble, afin de pouvoir polariser certaines électrodes (ou groupes d'électrodes) indépendamment les unes des autres. Une telle multipolarité permet notamment de disposer d'une fonction de "repositionnement électronique", laquelle offre de nombreuses possibilités de stimulation des tissus à travers le choix et la polarisation de certaines lignes de conduction parmi plusieurs incluses dans la microsonde, et de programmer spécifiquement des zones de stimulation ou de défibrillation en fonction de la thérapie.

Les Figures 7 et 8 illustrent un perfectionnement consistant à adjoindre à chaque microcâble un moyen de fixation à l'épicarde, au point d'émergence, au moyen d'une capsule d'ancrage 68 implantée localement en ce point (Figure 1).

Le rôle de cette capsule d'ancrage est double :
- dissocier la partie du microcâble implantée dans le myocarde du reste de la sonde, notamment pour éviter après implantation et avant la fin de l'intervention un déplacement du microcâble consécutif à une traction accidentelle exercée sur le corps de sonde ;
- gérer l'angulation de sortie (typiquement à angle droit) du microcâble à proximité du point d'émergence, en évitant les mouvements de forte amplitude autour de cette zone fortement courbée, donc contrainte (même si des boucles de compliance sont prévues dans la partie émergée de la microsonde pour absorber les contraintes et les mouvements entre le point d'émergence et le corps de sonde proprement dit).

La capsule d'ancrage 60 illustrée Figure 7 comprend un corps portant une fixation de type vis hélicoïdale 66. Dans un premier mode de mise en oeuvre, la capsule d'ancrage 64 est vissée à proximité immédiate du point d'émergence du microcâble après injection de ce dernier. La partie émergente du microcâble est alors enroulée sur une partie 68 de la capsule d'ancrage conformée en tambour d'enroulement, puis elle est clipsée dans une fente de sortie appropriée formée sur le corps de la capsule 64, en laissant un jeu minimum entre le point d'émergence et la capsule. Dans une autre mise en oeuvre possible, illustrée Figure 8, la capsule d'ancrage 64 est prémontée sur le corps de l'aiguille 42 dans la zone proximale de celle-ci. Une fois l'aiguille en position et le microcâble injecté dans le myocarde, la capsule est vissée puis l'aiguille est retirée. Le microcâble se trouve alors enserré dans le perçage axial de la capsule 64 où il reste maintenu en place malgré les sollicitations diverses qui pourront être appliquées à la partie émergente de la sonde.

D'autres moyens de maintien en place du microcâble à son point d'émergence peuvent être envisagés, parmi lesquels on peut notamment citer le dépôt d'un point de colle d'un adhésif chirurgical biocompatible tel que *Bioglue* (marque déposée) de la société Cryolife Inc., USA.

L'un des avantages majeurs de l'invention est la possibilité d'injecter les microcâbles par une manoeuvre chirurgicale peu invasive, typiquement une approche de type sub-xiphoïdienne, et en n'utilisant que des accessoires conventionnels.

Comme illustré Figure 9, l'embout 46 de l'aiguille 42 est monté sur un corps de seringue 70. Pendant l'injection, le microcâble est maintenu le long de l'aiguille et bloqué en translation par le clipsage dans le plot élastique 60, empêchant ainsi la libération du crochet formé par l'extrémité repliée 60. Cette configuration permet si besoin quelques mouvements l'aller-retour de l'aiguille afin d'affiner la trajectoire de celle-ci dans le muscle cardiaque.

L'extrémité de l'aiguille peut être dotée de moyens de repérage (marqueur radio-opaque, échographique, etc.) permettant de guider l'opérateur pendant la manoeuvre, afin notamment de s'assurer que la trajectoire choisie reste majoritairement dans le muscle. Il est également possible d'injecter du produit de contraste par la seringue à travers l'aiguille, dans le même but.

L'aiguille ayant atteint sa position finale, l'opérateur libère le microcâble du plot élastique 60 qui le clipsait, et tire l'aiguille. Le corps du microcâble s'oppose au mouvement de retour sous l'effet de la compression des tissus, libérant ainsi l'extrémité repliée 50 qui fixe alors, par effet de crochet, le microcâble de façon définitive.

La présence des crochets 50, 50' formés par la partie repliée de l'extrémité distale des microcâbles 36, 36' procure une bonne résistance à l'extraction au moment de la manoeuvre d'injection, puis sous l'effet des contraintes internes sous l'effet des battements cardiaques.

Si toutefois l'on souhaite extraire complètement le microcâble, un effort soutenu de traction externe aura pour effet de redresser la plicature, permettant ainsi le retrait du microcâble. Le système de l'invention peut donc être explanté aisément et sans dommages majeurs pour les tissus, contrairement à la quasi-totalité des systèmes connus de stimulation du ventricule gauche.

## Revendications

1. Un ensemble de sonde intracardiaque de stimulation du ventricule gauche, apte à être combiné avec un générateur d'un dispositif médical implantable actif pour la délivrance d'impulsions de stimulation et/ou de resynchronisation et/ou de défibrillation cardiaque, cet ensemble comportant :
- une sonde apte à être implantée dans les tissus d'une région interne du myocarde, par pénétration dans l'épaisseur de la paroi du septum interventriculaire (16) ou bien dans l'épaisseur de la paroi libre (32) du ventricule gauche, sous la surface et le long de cette paroi entre la région de l'apex et la région auriculaire,
cette sonde (34, 34') étant une microsonde constituée dans sa partie active, distale par un microcâble (36, 36') comportant un coeur électriquement conducteur revêtu d'une couche électriquement isolante, la partie active comprenant une série de zones dénudées (38, 38') du microcâble formant électrodes de stimulation électriquement reliées ensemble ; et
- un accessoire d'implantation de la sonde,
**caractérisé en ce que** :
- le diamètre du microcâble (36, 36') est d'au plus 1 French (0,33 mm) ;
- l'extrémité distale libre du microcâble est une extrémité repliée sur elle-même (50, 50') ;
- l'accessoire d'implantation (40) comporte une aiguille (42) avec une extrémité libre pointue de ponction (52) et une extrémité opposée montée sur un embout de préhension (46), cette aiguille étant au moins dans sa partie distale une aiguille creuse comportant une lumière interne débouchante (48) ;
- la partie repliée (50) de l'extrémité distale du microcâble est insérée dans la lumière interne de l'aiguille, et la partie non repliée du microcâble (36) s'étend contre la surface extérieure de l'aiguille le long de celle-ci jusqu'à l'embout de préhension ; et
- l'accessoire d'implantation (40) comporte en outre des moyens (60) libérables de maintien du microcâble, pour le support et la rétention du microcâble sur la longueur de l'aiguille entre l'extrémité de ponction et l'embout de préhension,
de manière à permettre l'implantation de la microsonde par pénétration simultanée de l'aiguille et du microcâble porté par cette aiguille, dans ladite épaisseur de la paroi du septum interventriculaire (16) ou de la paroi libre (32) du ventricule gauche,.

2. L'ensemble de la revendication 1, dans lequel la partie distale (44) de l'aiguille (42) comporte un canal (54) prolongeant la lumière interne (48) et apte à permettre la mise en place et le retrait de l'extrémité repliée (50) du microcâble (36).

3. L'ensemble de la revendication 1, dans lequel, à l'endroit du bord proximal (56) du canal, le débouché de la lumière centrale de l'aiguille n'est pas affuté, de manière à éviter tout endommagement de la partie repliée (50) du microcâble à l'endroit de la commissure (58) de celle-ci.

4. L'ensemble de la revendication 1, dans lequel la longueur de la partie active du microcâble (36) comprenant la série de zones dénudées (38) formant électrodes est comprise entre 50 et 150 mm.

5. L'ensemble de la revendication 1, dans lequel la longueur de la partie repliée de l'extrémité distale du microcâble est comprise entre 2 et 5 mm.

6. L'ensemble de la revendication 1, dans lequel l'embout (46) comprend un plot élastique (60) formant lesdits moyens libérables de maintien du microcâble, de manière à permettre le maintien du microcâble à l'état tendu entre le plot et le débouché (56) de la lumière interne de l'aiguille.

7. L'ensemble de la revendication 6, dans lequel le plot élastique (60) est un plot de matériau silicone comportant une fente longitudinale (62) de clipsage du microcâble.

8. L'ensemble de la revendication 1, dans lequel le coeur électriquement conducteur du microcâble (36) comprend une structure composite avec une pluralité de brins toronnés ensemble, dont au moins certains sont des brins incorporant une âme en matériau radio-opaque de type platine-iridium ou tantale enveloppée dans une gaine en matériau mécaniquement endurant de type NiTi ou alliage de type MP35NLT, ou inversement.

9. L'ensemble de la revendication 1, dans lequel le diamètre externe de l'aiguille (42) est compris entre 0,2 et 0,5 mm.

10. L'ensemble de la revendication 1, comprenant en outre des moyens (64) d'ancrage du microcâble à une surface du myocarde.

11. L'ensemble de la revendication 10, dans lequel les moyens d'ancrage comprennent une capsule (64) à vis hélicoïdale (66), pourvue de moyens de solidarisation au microcâble dans une région axiale de la capsule.

12. L'ensemble de la revendication 11, dans lequel la surface extérieure de la capsule à vis (64) est conformée en un tambour d'enroulement (68) apte à recevoir une longueur excédentaire du microcâble.

13. L'ensemble de la revendication 11, dans lequel la capsule à vis (64) comprend un organe élastique avec un perçage axial apte à recevoir, à proximité de l'embout de préhension, l'aiguille (42) pourvue du microcâble (36).

14. L'ensemble de la revendication 10, dans lequel les moyens d'ancrage comprennent un point de colle d'un adhésif chirurgical biocompatible.

15. L'ensemble de la revendication 1, dans lequel la surface totale exposée des zones dénudées (38) de la microsonde est d'au plus 6 mm².

## Patentansprüche

1. Anordnung einer intrakardialen Sonde zur Stimulation des linken Ventrikels, die geeignet ist, mit einem Generator eines aktiven implantierbaren medizinischen Geräts verbunden zu werden, zur Lieferung von Impulsen zur kardialen Stimulation und/oder Resynchronisation und/oder Defibrillation, wobei diese Anordnung aufweist:
- eine Sonde, die geeignet ist, in die Gewebe eines inneren Bereiches des Myokards durch Eindringen in die Dicke der Wand des interventrikulären Septums (16) oder auch in die Dicke der freien Wand (32) des linken Ventrikels, unter der Oberfläche und entlang dieser Wand zwischen dem Bereich des Apex und dem aurikulären Bereich, implantiert zu werden, wobei diese Sonde (34, 34') eine Mikrosonde ist, die in ihrem aktiven, distalen Teil aus einem Mikrokabel (36, 36') besteht, das einen elektrisch leitenden Kern aufweist, der von einer elektrisch isolierenden Schicht bedeckt ist, wobei der aktive Teil eine Reihe von abisolierten Bereichen (38, 38') des Mikrokabels aufweist, welche Stimulationselektroden bilden, die elektrisch miteinander verbunden sind; und
- ein Implantationszubehör der Sonde,
**dadurch gekennzeichnet, dass**:
- der Durchmesser des Mikrokabels (36, 36') höchstens 1 French (0,33 mm) beträgt;
- das freie distale Ende des Mikrokabels ein auf sich selbst zurückgefaltetes Ende (50, 50') ist;
- das Implantationszubehör (40) eine Nadel (42) mit einem freien zugespitzten Punktionsende (52) und einem gegenüberliegenden Ende, das an einem Greifendstück (46) angebracht ist, aufweist, wobei diese Nadel wenigstens in ihrem distalen Teil eine hohle Nadel ist, die ein nach außen mündendes Innenlumen (48) aufweist;
- der gefaltete Teil (50) des distalen Endes des Mikrokabels in das Innenlumen der Nadel eingesetzt ist und der nicht gefaltete Teil des Mikrokabels (36) sich, an der Außenseite der Nadel anliegend, entlang derselben bis zu dem Greifendstück erstreckt; und
- das Implantationszubehör (40) außerdem freigebbare Halterungsmittel (60) des Mikrokabels, die zum Abstützen und zum Halten des Mikrokabels auf der Länge der Nadel zwischen dem Punktionsende und dem Greifendstück bestimmt sind, aufweist,
derart, dass die Implantation der Mikrosonde durch gleichzeitiges Eindringen der Nadel und des von dieser Nadel getragenen Mikrokabels in die Dicke der Wand des interventrikulären Septums (16) oder der freien Wand (32) des linken Ventrikels ermöglicht wird.

2. Anordnung nach Anspruch 1, wobei der distale Teil (44) der Nadel (42) einen Kanal (54) aufweist, der das Innenlumen (48) verlängert und geeignet ist, die Anbringung und das Zurückziehen des gefalteten Endes (50) des Mikrokabels (36) zu ermöglichen.

3. Anordnung nach Anspruch 1, wobei am Ort des proximalen Randes (56) des Kanals die Mündung des zentralen Lumens der Nadel nicht zugespitzt ist, um jede Beschädigung des gefalteten Teils (50) des Mikrokabels am Ort des Zusammenpressens (58) desselben zu vermeiden.

4. Anordnung nach Anspruch 1, wobei die Länge des aktiven Teils des Mikrokabels (36), der die Reihe von abisolierten Bereichen (38) aufweist, welche Elektroden bilden, zwischen 50 und 150 mm liegt.

5. Anordnung nach Anspruch 1, wobei die Länge des gefalteten Teils des distalen Endes des Mikrokabels zwischen 2 und 5 mm liegt.

6. Anordnung nach Anspruch 1, wobei das Endstück (46) einen elastischen Klotz (60) aufweist, welcher die freigebbaren Halterungsmittel des Mikrokabels bildet, um das Halten des Mikrokabels im gespannten Zustand zwischen dem Klotz und der Mündung (56) des Innenlumens der Nadel zu ermöglichen.

7. Anordnung nach Anspruch 6, wobei der elastische Klotz (60) ein Klotz aus Silikonmaterial ist, der einen Längsschlitz (62) zum Einrasten des Mikrokabels aufweist.

8. Anordnung nach Anspruch 1, wobei der elektrisch leitende Kern des Mikrokabels (36) eine Verbundstruktur mit mehreren miteinander verdrillten Adern aufweist, von denen wenigstens einige Adern sind, die eine Seele aus einem strahlenundurchlässigen Material vom Typ Platin-Iridium oder Tantal enthalten, die von einem Mantel aus einem mechanisch beständigen Material vom Typ NiTi oder einer Legierung vom Typ MP35NLT umgeben ist, oder umgekehrt.

9. Anordnung nach Anspruch 1, wobei der Außendurchmesser der Nadel (42) zwischen 0,2 und 0,5 mm liegt.

10. Anordnung nach Anspruch 1, welche außerdem Mittel (64) zur Verankerung des Mikrokabels an einer Fläche des Myokards aufweist.

11. Anordnung nach Anspruch 10, wobei die Mittel zur Verankerung eine Kapsel (64) mit einer Schneckenwendel (66) aufweisen, die mit Mitteln zur festen Verbindung mit dem Mikrokabel in einem axialen Bereich der Kapsel versehen ist.

12. Anordnung nach Anspruch 11, wobei die Außenfläche der Schraubkapsel (64) als eine Aufwickeltrommel (68) ausgebildet ist, die geeignet ist, eine überschüssige Länge des Mikrokabels aufzunehmen.

13. Anordnung nach Anspruch 11, wobei die Schraubkapsel (64) ein elastisches Organ mit einer axialen Bohrung aufweist, die geeignet ist, in der Nähe des Greifendstücks die mit dem Mikrokabel (36) versehene Nadel (42) aufzunehmen.

14. Anordnung nach Anspruch 10, wobei die Mittel zur Verankerung einen Klebepunkt eines biokompatiblen chirurgischen Klebstoffs aufweisen.

15. Anordnung nach Anspruch 1, wobei die freiliegende Gesamtfläche der abisolierten Bereiche (38) der Mikrosonde höchstens 6 mm² beträgt.

## Claims

1. An endocardial lead system for stimulation of the left ventricle, adapted to be combined with a generator of an active implantable medical device for the delivery of cardiac stimulation and/or resynchronisation and/or defibrillation pulses, this system including:
- a lead adapted to be implanted in the tissues of an inner region of the myocardium, by penetration in the thickness of the wall of the interventricular septum (16) or in the thickness of the free wall (32) of the left ventricle, under the surface and along this wall between the apical region and the atrial region,
this lead (34, 34') being a micro-lead consisted, in its distal active part, by a micro-cable (36, 36') including an electrically conductive core coated with an electrically insulating layer, the active part comprising a series of naked zones (38, 38') of the micro-cable, forming stimulation electrodes that are electrically connected together; and
- an accessory for implantation of the lead,
**characterized in that**:
- the diameter of the micro-cable (36, 36') is at most of 1 French (0.33 mm);
- the free distal end of the micro-cable is an end (50, 50') that is folded over itself;
- the implantation accessory (40) includes a needle (42) with a free acute puncturing end (52) and an opposite end mounted on a gripping tip (46), this needle being, at least in its distal part, a hollow needle including an inner through-lumen (48);
- the folded part (50) of the distal end of the micro-cable is inserted in the inner lumen of the needle, and the non-folded part of the micro-cable (36) extends against the outer surface of the needle, along the latter, up to the gripping tip; and
- the implantation accessory (40) further includes releasable means (60) for maintaining the micro-cable, for the support and the holding of the micro-cable over the length of the needle between the puncturing end and the gripping tip,
so as to allow the implantation of the micro-lead by simultaneous penetration of the needle and of the micro-cable carried by this needle in said thickness of the wall of the interventricular septum (16) or of the free wall (32) of the left ventricle.

2. The system of claim 1, wherein the distal part (44) of the needle (42) includes a channel (54) continuing the inner lumen (48) and adapted to allow the installation and the removal of the folded end (50) of the micro-cable (36) .

3. The system of claim 1, wherein, at the location of the proximal edge (56) of the channel, the opening of the central lumen of the needle is not sharpened, so as to avoid any damage of the folded part (50) of the micro-cable at the location of the commissure (58) thereof.

4. The system of claim 1, wherein the length of the active part of the micro-cable (36) comprising the series of naked areas (38) forming electrodes is comprised between 50 and 150 mm.

5. The system of claim 1, wherein the length of the folded part of the distal end of the micro-cable is comprised between 2 and 5 mm.

6. The system of claim 1, wherein the tip (46) comprises an elastic stud (60) forming said releasable means for maintaining the micro-cable, so as to allow the holding of the micro-cable in the tight state between the stud and the opening (56) of the inner lumen of the needle.

7. The system of claim 6, wherein the elastic stud (60) is a silicone material stud including a longitudinal slit (62) for the quick connection of the micro-cable.

8. The system of claim 1, wherein the electrically conductive core of the micro-cable (36) comprises a composite structure with a plurality of wires stranded together, at least some of which are wires incorporating a core made of a radio-opaque material of the platinum-iridium or tantalum type enveloped with a sheath made of a mechanically tough material of the NiTi type or an alloy of the MP35NLT type, or the reverse.

9. The system of claim 1, wherein the outer diameter of the needle (42) is comprised between 0.2 and 0.5 mm.

10. The system of claim 1, further comprising means (64) for the anchoring of the micro-cable to a surface of the myocardium.

11. The system of claim 10, wherein the anchoring means comprise a capsule (64) with an helical screw (66), provided with means for the fastening to the micro-cable in an axial region of the capsule.

12. The system of claim 11, wherein the outer surface of the screw capsule (64) is shaped as a winding drum (68) adapted to receive an excess length of the micro-cable.

13. The system of claim 11, wherein the screw capsule (64) comprises an elastic member with an axial drill adapted to receive, near the gripping tip, the needle (42) provided with the micro-cable (36).

14. The system of claim 10, wherein the anchoring means comprise a point of glue consisted of a biocompatible surgical adhesive.

15. The system of claim 1, wherein the total exposed surface of the naked areas (38) of the micro-cable is at most of 6 mm².
